# EUROPEAN PATENT APPLICATION

(11) **EP 4 310 724 A1**
(43) Date of publication of application: **24.01.2024**
(21) Application number: 22783901.6
(22) Date of filing: 28.03.2022
(51) Int. Cl.: G06K 9/00

(54) **METHOD FOR DETERMINING EXERCISE GUIDANCE INFORMATION, ELECTRONIC DEVICE, AND EXERCISE GUIDANCE SYSTEM**

(30) Priority: 07.04.2021 CN 202110374190
(71) Applicant: Huawei Technologies Co., Ltd., Shenzhen, Guangdong 518129 (CN)
(72) Inventor: MA, Shanshan, Shenzhen, Guangdong 518129 (CN); LI, Junjin, Shenzhen, Guangdong 518129 (CN); JIANG, Yonghang, Shenzhen, Guangdong 518129 (CN); LIU, Hang, Shenzhen, Guangdong 518129 (CN)
(74) Representative: Huawei European IPR
(86) International application number: PCT/CN2022/083336
(87) International publication number: WO 2022/213834

(57) **Abstract**

This application provides a method for determining exercise guidance information, an electronic device, and an exercise guidance system, and relates to the field of human-computer interaction technologies. The method is applied to an electronic device, and includes: displaying a first preset image and a captured user image; recognizing a deflection angle of a virtual user in the user image; determining, from a demonstration action set, a second preset image corresponding to the deflection angle, where preset images corresponding to different deflection angles are stored in the demonstration action set; and determining exercise guidance information for a user based on the user image and the second preset image, where the first preset image is the same as or different from the second preset image. According to the technical solutions provided in embodiments of this application, accuracy of user action recognition can be improved. In a process of doing exercise, the user does not need to be limited by the deflection angle, and can select, based on a preference of the user, a deflection angle relative to the electronic device. This provides better user experience.

## Description

This application claims priority to Chinese Patent Application No. 202110374190.3, filed with the China National Intellectual Property Administration on April 7, 2021 and entitled "METHOD FOR DETERMINING EXERCISE GUIDANCE INFORMATION, ELECTRONIC DEVICE, AND EXERCISE GUIDANCE SYSTEM", which is incorporated herein by reference in its entirety.

### TECHNICAL FIELD

This application relates to the field of human-computer interaction technologies, and in particular, to a method for determining exercise guidance information, an electronic device, and an exercise guidance system.

### BACKGROUND

With development of computer technologies, an electronic device (for example, a smart television) equipped with a camera can guide a user to do exercise, for example, guide the user to work out, do yoga, dance, do Tai Chi, and play basketball. An example in which the electronic device guides the user to work out is used. The electronic device displays a preset demonstration image for the user to imitate and learn, captures a user image through a camera, to recognize a human body posture of the user, and provides guidance on a workout action of the user based on the human body posture.

However, in a process of doing an action based on the demonstration image, the user needs to face the electronic device at a specific angle. For example, when the demonstration image displayed by the electronic device is a front-side exercise image of a demonstrator, a front side of a body of the user needs to face the electronic device. Alternatively, when the demonstration image displayed by the electronic device is a lateral-side exercise image of the demonstrator, a lateral side of the body of the user needs to face the electronic device. Otherwise, the electronic device cannot recognize or accurately recognize a user action, and user experience is poor.

### SUMMARY

Embodiments of this application provide a method for determining exercise guidance information, an electronic device, and an exercise guidance system, to improve accuracy of recognizing a user action by the electronic device.

To achieve the foregoing objective, the following technical solutions are used in this application:
According to a first aspect, an embodiment of this application provides a method for determining exercise guidance information, applied to an electronic device. The method includes: displaying a first preset image and a captured user image; recognizing a deflection angle of a virtual user in the user image; determining, from a demonstration action set, a second preset image corresponding to the deflection angle, where preset images corresponding to different deflection angles are stored in the demonstration action set; and determining exercise guidance information for a user based on the user image and the second preset image, where the first preset image is the same as or different from the second preset image.

In the method provided in this embodiment of this application, after determining the deflection angle of the user image, the electronic device compares the user image with the second preset image corresponding to the deflection angle, to determine the exercise guidance information for the user. According to the method, accuracy of user action recognition can be improved. On this basis, in a process of doing exercise, the user does not need to be limited by the deflection angle, and can select, based on a preference of the user, a deflection angle relative to the electronic device. This provides better user experience.

In some embodiments, the method further includes: displaying the second preset image while displaying the first preset image; or replacing the first preset image with the second preset image. According to the method provided in this embodiment, the user may observe, on the electronic device, the preset image corresponding to the deflection angle of the user image.

In some embodiments, the exercise guidance information includes at least one of an action score, an action comment, an action evaluation icon, posture correction information, consumed calories, and exercise duration.

According to a second aspect, an embodiment of this application provides an electronic device. The electronic device is configured to: display a first preset image and a captured user image; recognize a deflection angle of a virtual user in the user image; determine, from a demonstration action set, a second preset image corresponding to the deflection angle, where preset images corresponding to different deflection angles are stored in the demonstration action set; and determine exercise guidance information for a user based on the user image and the second preset image, where the first preset image is the same as or different from the second preset image.

In some embodiments, the electronic device is further configured to: display the second preset image while displaying the first preset image; or replace the first preset image with the second preset image.

In some embodiments, the exercise guidance information includes at least one of an action score, an action comment, an action evaluation icon, posture correction information, consumed calories, and exercise duration.

According to a third aspect, an embodiment of this application provides an exercise guidance system. The exercise guidance system includes a first electronic device and at least one second electronic device. The first electronic device is configured to perform the method shown in the first aspect. The first electronic device determines exercise guidance information for a user based on both locally collected user data and user data collected by the at least one second electronic device. The at least one second electronic device is the same or different.

According to the system provided in this embodiment of this application, on the basis of determining a corresponding second preset image based on a deflection angle of a virtual user, and further recognizing a user action based on the second preset image, the exercise guidance information for the user can be further jointly determined with reference to multi-dimensional data obtained from a plurality of sources. This improves accuracy of exercise guidance.

In some embodiments, the user data collected by the first electronic device is a first user image, the user data collected by the second electronic device is a second user image, and a deflection angle of a virtual user in the first user image is different from that in the second user image.

In some embodiments, the user data collected by the second electronic device is body fat information; and the first electronic device recommends a corresponding exercise item to the user based on the body fat information. The system provided in this embodiment can recommend an exercise item that is suitable for the user to the user. For example, when a body fat percentage of the legs of the user exceeds a standard, a leg fat reduction item is recommended to the user. When a body fat percentage of the waist and the abdomen of the user exceeds a standard, a waist and abdomen fat reduction item is recommended to the user.

In some embodiments, the user data collected by the second electronic device is a personal physical sign parameter; and the first electronic device displays health prompt information based on the personal physical sign parameter, where the health prompt information indicates whether the user can do exercise.

In some embodiments, the personal physical sign parameter includes at least one of a heart rate, blood pressure, body temperature, and blood oxygen saturation.

In some embodiments, the user data collected by the second electronic device includes a jump height and a jump frequency of the user.

In some embodiments, the first electronic device is connected to the at least one second electronic device through a far field communication technology, a near field communication technology, or a physical entity.

According to a fourth aspect, an embodiment of this application provides a chip system. The chip system includes a memory and a processor. The processor executes a computer program stored in the memory, to implement the method shown in the first aspect. The chip system may be a single chip or a chip module including a plurality of chips.

According to a fifth aspect, an embodiment of this application provides a computer-readable storage medium. The computer-readable storage medium stores a computer program. When the computer program is executed by a processor, the method shown in the first aspect is implemented.

It may be understood that, for beneficial effects of the second aspect, the fourth aspect, and the fifth aspect, refer to related descriptions in the first aspect. Details are not described herein again.

### BRIEF DESCRIPTION OF DRAWINGS

FIG. 1A is a schematic diagram 1 of an exercise guidance scenario of an electronic device according to an embodiment of this application;
FIG. 1B is a schematic diagram 2 of an exercise guidance scenario of an electronic device according to an embodiment of this application;
FIG. 2 is a schematic flowchart of a method for determining exercise guidance information according to an embodiment of this application;
FIG. 3 is a schematic diagram of a deflection angle according to an embodiment of this application;
FIG. 4 is a schematic diagram of a process of determining a deflection angle according to an embodiment of this application;
FIG. 5A is a schematic diagram 1 of interface display of an electronic device according to an embodiment of this application;
FIG. 5B is a schematic diagram 2 of interface display of an electronic device according to an embodiment of this application;
FIG. 6 is a schematic diagram of an application scenario of an exercise guidance system according to an embodiment of this application;
FIG. 7 is a schematic diagram of an image capture scenario of a large-screen device according to an embodiment of this application;
FIG. 8A is a schematic diagram 1 of user data processing according to an embodiment of this application;
FIG. 8B is a schematic diagram 2 of user data processing according to an embodiment of this application;
FIG. 8C is a schematic diagram 3 of user data processing according to an embodiment of this application;
FIG. 9 is a schematic flowchart of selecting a central computing device according to an embodiment of this application;
FIG. 10 is a schematic diagram of computing a height of a user according to an embodiment of this application;
FIG. 11 is a schematic diagram of user data processing according to an embodiment of this application;
FIG. 12A is a schematic diagram of a capture scenario of a first user image according to an embodiment of this application;
FIG. 12B is a schematic diagram of a capture scenario of a second user image according to an embodiment of this application;
FIG. 12C is a schematic diagram of a capture scenario of a third user image according to an embodiment of this application;
FIG. 13 is a schematic diagram of computing a jump height of a user according to an embodiment of this application;
FIG. 14 is a schematic diagram of a scoring interface according to an embodiment of this application; and
FIG. 15 is a schematic diagram of a structure of an electronic device according to an embodiment of this application.

### DESCRIPTION OF EMBODIMENTS

The following describes technical solutions provided in embodiments of this application with reference to the accompanying drawings.

In this embodiment, an electronic device may be a terminal device equipped with a camera, such as a large-screen device (for example, a smart television, a smart screen, or a projector), a mobile phone, a tablet computer, a notebook computer, a desktop computer, a smart television, an ultra-mobile personal computer (ultra-mobile personal computer, UMPC), a netbook, or a personal digital assistant (personal digital assistant, PDA).

With development of computer technologies, an electronic device (for example, a smart television or a mobile phone) can guide a user to do exercise, for example, guide the user to do artificial intelligence (artificial intelligence, AI) workout, do yoga, dance, do Tai Chi, and play basketball. An example in which the electronic device guides the user to do AI workout is used. In a process of guiding the user to do exercise, the electronic device displays a demonstration image for the user to imitate and learn a demonstration action. In addition, the electronic device further captures and displays a user image, compares the user image with the demonstration image displayed by the electronic device, to recognize a human body posture of the user, determines a completion status of the demonstration action performed by the user, and generates exercise guidance information. For example, the exercise guidance information may be an action score, an action comment, an action evaluation icon, posture correction information, consumed calories, exercise duration, or the like.

On this basis, currently, the user needs to face the electronic device at a specific angle, to ensure that the user image and the demonstration image are images at a same angle, so that the electronic device can accurately recognize the user action. However, when the user does not face the electronic device at the specific angle, the user image captured by the electronic device and a preset demonstration image are not images at the same angle. In this case, even if the user actually performs the demonstration action accurately, because there is a great difference between the user image and the demonstration image, the electronic device cannot accurately recognize the user action, resulting in poor user experience.

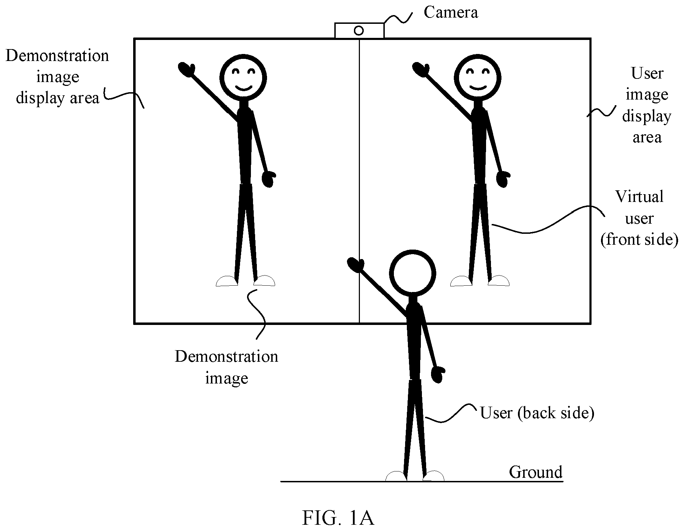

For example, as shown in FIG. 1A, when the demonstration image displayed by the electronic device is a front-side exercise image of a demonstrator, a front side of a body of the user faces the electronic device, and both the demonstration image and the user image are images at the same angle (namely, front-side images). The electronic device compares the front-side user image with the front-side demonstration image, to accurately recognize an action of the user and output relatively accurate exercise guidance information.

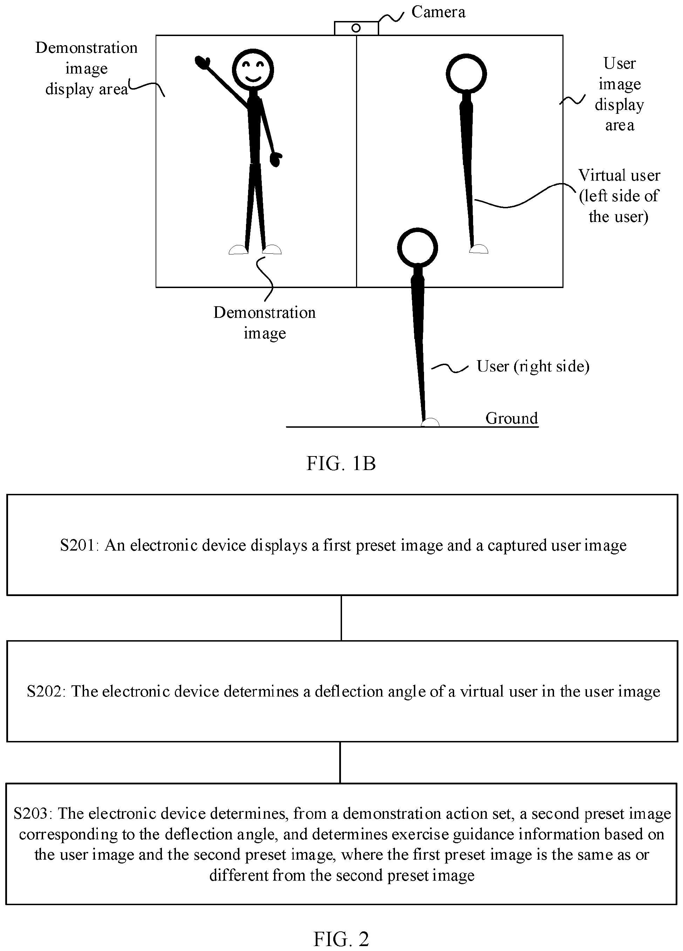

Otherwise, as shown in FIG. 1B, when the demonstration image displayed by the electronic device is a front-side exercise image of a demonstrator, if the left side of the body of the user faces the electronic device, the user image captured by the electronic device is an image of the left side of the body of the user. In this case, even if the user actually completes the demonstration action accurately, because the user image and the demonstration image are not images at the same angle, and there is a great difference between the user image and the demonstration image, the electronic device cannot accurately recognize the user action when comparing the user image with the demonstration image, even considers that the user does not complete the demonstration action, and outputs inaccurate exercise guidance information, resulting in poor user experience.

Therefore, this embodiment of this application provides a method for determining exercise guidance information, so that accuracy of recognizing a user action by an electronic device can be improved, and a user can be free from being constrained by the foregoing specific angle in an exercise process, and can freely select an angle to face the electronic device. This improves user experience.

The following uses an example in which the electronic device is a large-screen device to describe, with reference to a scenario in which the electronic device guides the user to work out, the method for determining exercise guidance information provided in this embodiment of this application. It should be understood that the technical solution provided in this embodiment is further applicable to other exercise scenarios based on human-computer interaction, such as dancing, doing Tai Chi, playing basketball, and playing a motion sensing game.

FIG. 2 is a schematic flowchart of a method for determining exercise guidance information according to an embodiment of this application. FIG. 2 shows a process in which an electronic device intelligently recognizes a user action based on a deflection angle of a virtual user in a user image, to determine the exercise guidance information. Specifically, the following steps S201 to S203 are included.

S201: An electronic device displays a first preset image and a captured user image.

The electronic device guides, by running an exercise application (application, App), a user to do exercise. The electronic device can provide various different exercise items based on a pre-configuration of the app. For example, the exercise items that can be provided by an AI workout application may include a "shoulder and neck circle exercise", a "chest expansion exercise", a "stretching exercise", a "kicking exercise", and the like. In this embodiment, an exercise item (for example, the "shoulder and neck circle exercise") is a sub-application set in an application that provides an exercise guidance service. One application usually includes a plurality of sub-applications. The electronic device can provide different guidance content by running different sub-applications.

In this embodiment, for each exercise item, a demonstration action set is preset in the application. The demonstration action set includes a plurality of groups of demonstration images, and a demonstrator in each group of demonstration images has different deflection angles, for example, a front-side image, a back-side image, or a left-side image of the demonstrator. Demonstration images corresponding to different deflection angles can present demonstration actions from different angles for the user to imitate and learn.

It should be noted that the demonstrator is a virtual character displayed in a demonstration action display area of the electronic device, and is configured to display a preset demonstration action. The user is a real character who participates in an exercise item. The virtual user is the virtual character in the user image captured by the electronic device. It should be understood that, in a process of capturing, by the electronic device, the user image in real time, the user and the virtual user usually have a mirror symmetry relationship.

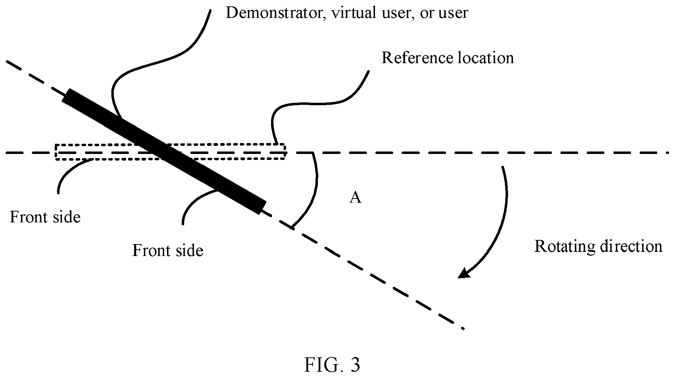

In this embodiment, as shown in FIG. 3, the deflection angle is an angle by which a rotating object needs to rotate clockwise or counterclockwise from a reference location to a current location by using a body as a central axis. When the rotating object rotates clockwise, the deflection angle is positive, and ranges from 0° to 360°; and when the rotating object rotates counterclockwise, the deflection angle is negative, and ranges from 0° to -360°. For the demonstration image and the user image, when the front side of the demonstrator or the virtual user is displayed on a screen of the electronic device, a location at which the demonstrator or the virtual user is located is the reference location. For the user, a location at which the user is located when the front side of the user faces the screen of the electronic device is the reference location.

It should be understood that, because the virtual user and the user usually have the mirror symmetry relationship, a deflection angle of the virtual user is usually opposite to a deflection angle of the user. For example, as shown in FIG. 1A, when the actual user faces the electronic device and stands with a left hand raised, in the user image captured by the electronic device, the virtual user raises a right hand. When the user rotates clockwise, the virtual user rotates counterclockwise.

For a same demonstration action, when the demonstrator has a deflection angle, display effect of the action performed by the demonstrator is usually different. For example, for a "shoulder and neck circle action", a demonstration image in which a deflection angle of the demonstrator is 0° (that is, a demonstration image of the front side of the demonstrator) usually has good display effect, and actions of left and right arms may be simultaneously displayed. However, in a demonstration image in which a deflection angle of the demonstrator is 90° (that is, a demonstration image of a left side of the demonstrator), the right arm of the demonstrator is blocked, and the electronic device cannot clearly display an action of the right arm.

On this basis, in some embodiments, for each demonstration action, an app developer may preset, based on an action feature, one or more high-priority deflection angles. When displaying a demonstration image, the electronic device may display a demonstration image corresponding to the high-priority deflection angle as a first preset image. For example, for the "shoulder and neck circle action", the demonstration image in which the deflection angle is 0° (that is, the demonstration image of the front side of the demonstrator) is used as the first preset image, and is displayed on the screen of the electronic device, to clearly display the demonstration action to the user.

In some embodiments, the electronic device may alternatively determine and display the first preset image based on a user instruction. For example, the demonstration image in which the deflection angle of the demonstrator is 90° (that is, the demonstration image of the left side of the demonstrator) is displayed based on the user instruction. Alternatively, the demonstration image in which the deflection angle is 0° (that is, the demonstration image of the front side of the demonstrator) and the demonstration image in which the deflection angle of 90° (that is, the demonstration image of the left side of the demonstrator) are simultaneously displayed based on the user instruction.

S202: The electronic device determines the deflection angle of the virtual user in the user image.

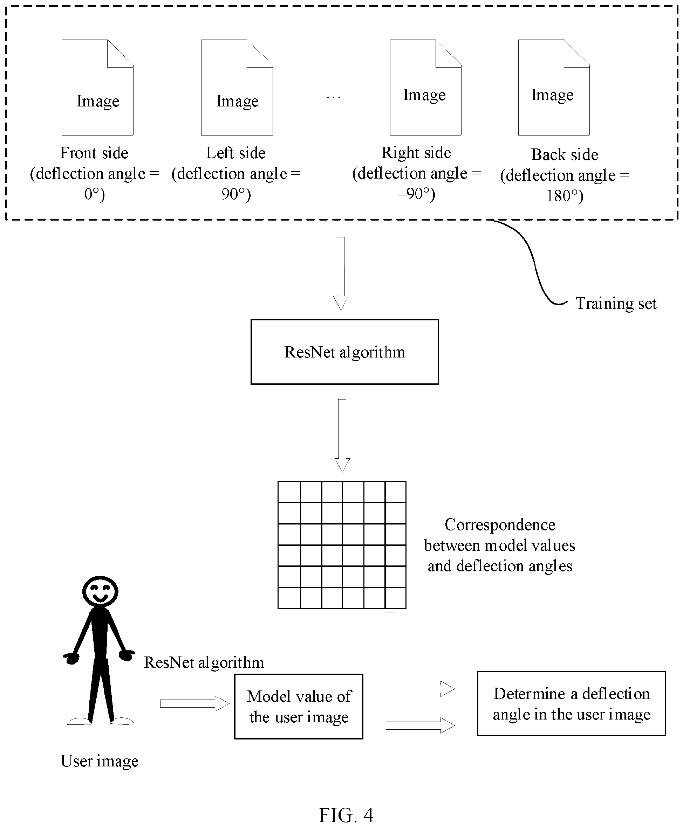

In this embodiment, the electronic device may compute the deflection angle of the virtual user in the user image according to a residual neural network (Residual Neural Network, ResNet) algorithm. For example, as shown in FIG. 4, the electronic device may obtain a training set in advance. Images in the training set include characters with different deflection angles and marking results of the deflection angles, for example, characters on the front side (deflection angle = 0°), left side (deflection angle = 90°), right side (deflection angle = -90°), and back side (deflection angle = 180°), and the corresponding marking results. The electronic device may learn the character images in the training set according to the ResNet algorithm, to determine a series of algorithm model values (namely, Model values). Each model value corresponds to one deflection angle. After capturing the user image, the electronic device may compute a model value of the user image according to the ResNet algorithm, to determine the deflection angle of the virtual user based on a correspondence between model values and deflection angles. Alternatively, the electronic device may recognize the deflection angle of the virtual user in the user image based on a preset deflection angle recognition model. This is not limited in this embodiment.

S203: The electronic device determines, from the demonstration action set, a second preset image corresponding to the deflection angle of the virtual user, and determines exercise guidance information based on the user image and the second preset image. The first preset image is the same as or different from the second preset image.

For example, as shown in FIG. 1B, when the demonstration image displayed by the electronic device is the front-side image of the demonstrator, if the electronic device detects that the deflection angle of the virtual user in the user image is -90° (in this case, a left side of the actual user faces the screen of the electronic device, the electronic device captures a left-side image of the user, and a right side of the virtual user is displayed on the electronic device), the second preset image, that is, a demonstration image in which a deflection angle of the demonstrator is -90° (a right-side image of the demonstrator) may be determined from a demonstration image set. Then, the electronic device compares the demonstration image in which the deflection angle of the demonstrator is -90° with the user image in which the deflection angle of the virtual user is -90°, to recognize an action of the user and determine the exercise guidance information.

In addition, optionally, after determining the deflection angle of the virtual user in the user image, the electronic device may alternatively display, in a demonstration image display area, a demonstration image corresponding to the deflection angle (namely, the second preset image).

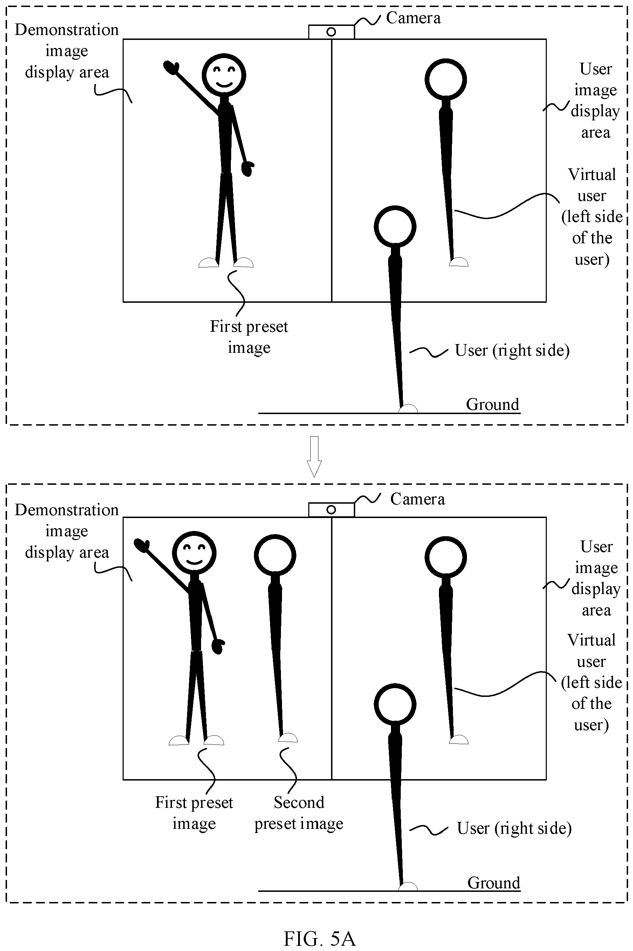

In some embodiments, the electronic device may simultaneously display the first preset image and the second preset image. For example, as shown in FIG. 5A, when the first preset image displayed by the electronic device is the front-side image of the demonstrator, if it is detected that the deflection angle of the virtual user is -90°, the demonstration image in which the deflection angle of the demonstrator is -90° may be used as the second preset image, and the second preset image and the first preset image (namely, the front-side image of the demonstrator) are simultaneously displayed on the electronic device.

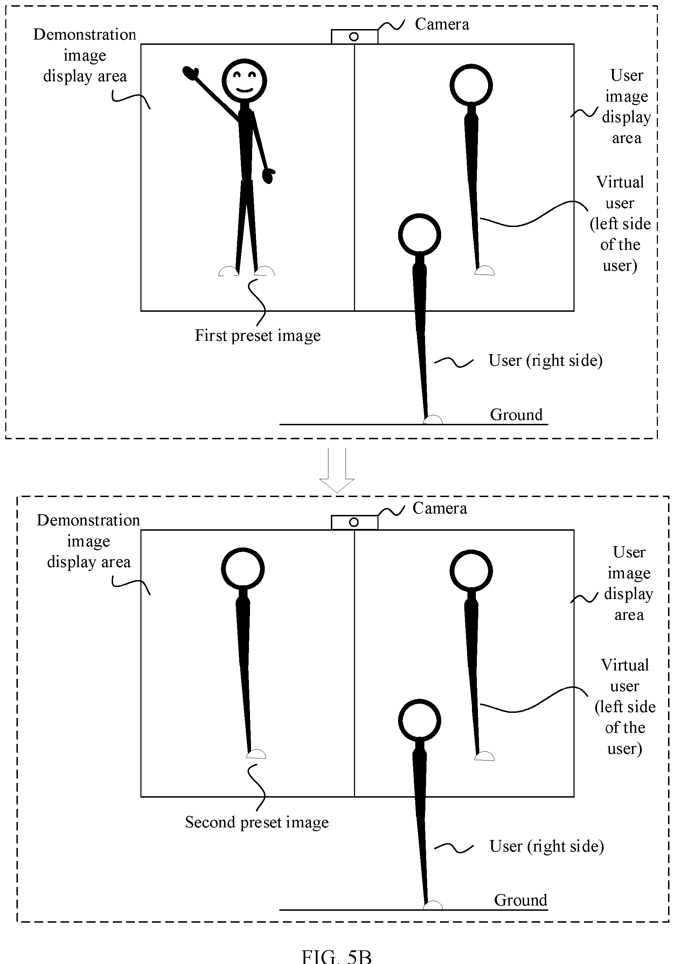

In some other embodiments, the electronic device may replace the first preset image with the second preset image. For example, as shown in FIG. 5B, when the first preset image displayed by the electronic device is the front-side image of the demonstrator, if it is detected that the deflection angle of the virtual user is -90°, the demonstration image in which the deflection angle of the demonstrator is -90° may be displayed on the electronic device as the second preset image, and the first preset image (namely, the front-side image of the demonstrator) is no longer displayed.

In addition, the user may change a deflection angle relative to the electronic device in a process of doing exercise, and consequently, the deflection angle of the virtual user also changes accordingly. Therefore, in a process of displaying the second preset image by the electronic device, if the electronic device detects that the deflection angle of the virtual user changes, the second preset image is updated again based on the changed deflection angle.

In conclusion, in this embodiment, even if presentation angles of an action are different in the user image and the first preset image displayed by the electronic device, the electronic device can still accurately recognize the user action. According to the method provided in this embodiment, in the process of doing exercise, the user does not need to be limited by the deflection angle, and can select, based on a preference of the user, the deflection angle relative to the electronic device. This provides better user experience.

Based on the electronic device provided in the foregoing embodiments of this application, an embodiment of this application further provides an exercise guidance system, configured to: in a process of guiding a user to do exercise, recognize a user action in a plurality of dimensions through a plurality of electronic devices, to more accurately determine exercise guidance information for the user.

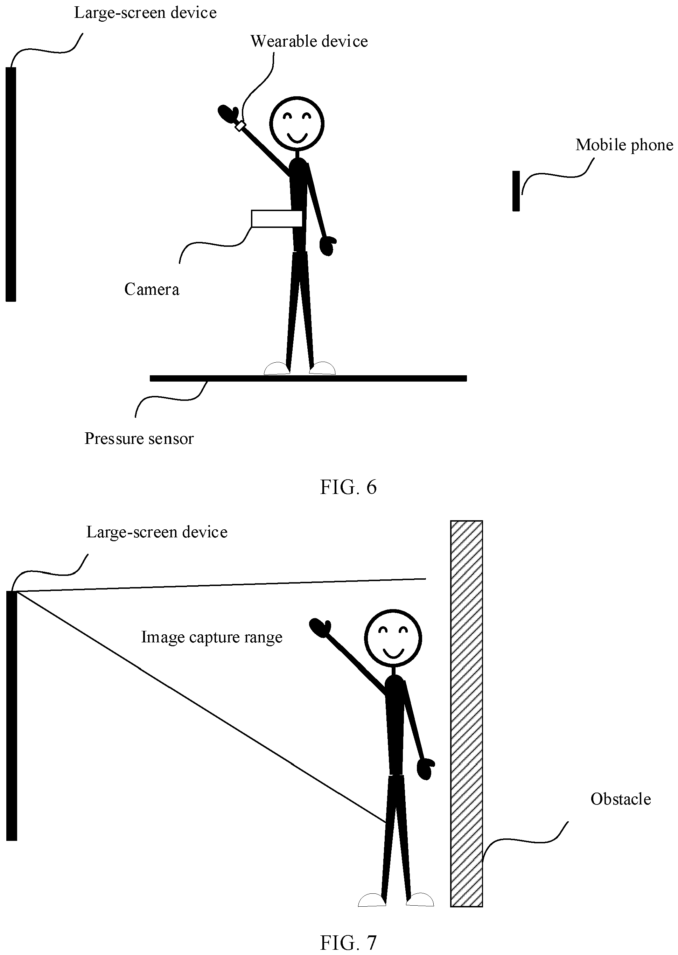

FIG. 6 is an application scenario of an exercise guidance system according to an embodiment of this application. As shown in FIG. 6, the system includes at least one electronic device provided in the foregoing embodiments of this application, for example, a large-screen device or a mobile phone. In addition, another electronic device such as a wearable device, a sensor device, or a camera may be further included.

In this embodiment, the wearable device may be a smartwatch, a smart band, a wearable wrist device, smart glasses, an augmented reality (augmented reality, AR)/virtual reality (virtual reality, VR) device, or the like.

It should be understood that, in this embodiment of this application, the wearable device may be alternatively a general term for wearable devices that are intelligently designed and developed for daily wear by using a wearable technology, for example, glasses, gloves, watches, clothes, and shoes. The wearable device is a portable device that is worn on a body directly or integrated into clothes or an accessory of a user. The wearable device is not only a hardware device, but also implements a powerful function through software support, data exchange, and cloud interaction. In a broad sense, wearable intelligent devices include full-featured and large-sized devices that can implement all or some of functions without relying on smartphones, for example, smart watches or smart glasses, and include devices that focus only on a specific type of application function and need to be used together with other devices such as smartphones, for example, various smart bands or smart jewelry for monitoring physical signs.

In this embodiment, the sensor device may be a ground mat, a wearable device, or the like on which a sensor is installed. The sensor may be a pressure sensor, an acceleration sensor, a gyroscope sensor, or the like. This is not limited in this embodiment.

The pressure sensor is configured to sense a pressure signal, and can convert the pressure signal into an electrical signal. In some embodiments, the pressure sensor may be disposed inside a ground mat such as a dancing blanket or a yoga mat. There are a plurality of types of pressure sensors such as a resistive pressure sensor, an inductive pressure sensor, and a capacitive pressure sensor. The capacitive pressure sensor may include at least two parallel plates made of conductive materials. When a force is applied to the pressure sensor, capacitance between electrodes changes, and the electronic device connected to the pressure sensor determines pressure intensity based on a capacitance change. When a pressure is applied to the ground mat, the electronic device connected to the ground mat may compute a location at which the pressure is applied based on the pressure intensity detected by the pressure sensor.

The acceleration sensor (acceleration, ACC) may be installed on a wearable device. When the user wears the wearable device on an arm to exercise, the wearable device may be configured to detect magnitudes of accelerations of the arm of the user in various directions (generally three axes), and can be used as a user action collector to sense a movement action of the arm of the user in a direction such as front and back, left and right, and up and down, and convert the movement action into a virtual scene action such as punching, swinging a racket, jumping, or throwing a fishing rod in a game scenario, and the like.

The gyroscope sensor may be configured to determine a motion posture of the electronic device. In some embodiments, angular velocities of the electronic device around three axes (namely, axes x, y, and z) may be determined by using the gyroscope sensor. The gyroscope sensor 280B may be used in image stabilization during photographing, navigation, and motion sensing games.

In this embodiment, the camera may be a component of an electronic device, or may be an independent electronic device. This is not limited in this embodiment.

The following describes a running process of the exercise guidance system provided in this embodiment.

It is a continuous process for the exercise guidance system to guide the user to do exercise. In this process, each electronic device in the exercise guidance system needs to continuously display related exercise information and collect user data.

Because a large-screen device (for example, a smart television) has a large display screen, the large-screen device is usually used to install an app that provides an exercise guidance service. After turning on an exercise item in the app based on a user operation, the large-screen device displays a first preset image corresponding to the exercise item and a locally captured first user image. For details, refer to the foregoing description. Details are not described herein in this embodiment.

The mobile phone is configured to capture a second user image. Specifically, based on a feature that the mobile phone is easy to move and place, the user may place the mobile phone at another location different from that of the large-screen device (for example, a location opposite to the large-screen device) based on a requirement, to capture the second user image from an angle different from that of the large-screen device.

Compared with the large-screen device, the mobile phone has a stronger image capture capability. For example, as shown in FIG. 7, a camera of the large-screen device usually does not have a zoom capability, and an image capture range of the camera is usually a fixed area. When the user is blocked by an obstacle (for example, a wall or a sofa in a living room) and movement space is insufficient, the camera of the large-screen device may capture only a partial image of the user, resulting in failure to recognize a user action. Different from the large-screen device, the mobile phone usually has an automatic zoom capability. A focal length of a lens in a camera may be automatically adjusted to change an image capture range, to capture a complete user image. This resolves, to some extent, a problem that large space needs to be reserved when the large-screen device is used to guide the user to do exercise. Optionally, in the system provided in this embodiment, the mobile phone may be controlled to increase a quantity of second user images to be captured, and the large-screen device may be controlled to reduce a quantity of first user images to be captured, to improve capture quality of user images.

The wearable device (for example, a smartwatch) may detect personal physical sign parameters of the user, such as a heart rate (heart rate, HR), blood pressure, body temperature, and blood oxygen saturation. In addition, when the user wears the wearable device to exercise, the wearable device can further record a movement track, an action amplitude, and the like of a wearing part (for example, a wrist or an ankle) of the user. In addition, a shake status, an exercise intensity, and the like of the wearing part in an exercise process are recorded. The shake status may be used to determine stability of the user action. The exercise intensity is used to indicate intensity of the exercise of the user.

The pressure sensor device (for example, a ground mat with a pressure sensor) may be configured to detect parameters such as a distance between two feet of the user, a weight of the user, a quantity of foot jumps, and a jump frequency.

It should be noted that, in this embodiment, when only the two feet of the user are in contact with the pressure sensor device, a distance between two pressure action points detected by the pressure sensor device is the distance between the two feet of the user. In a jump process of the user, the two feet of the user repeatedly perform an action of treading on the ground, and the pressure sensor device may count a quantity of jumps of the user based on a change status of treading pressure of the user. The jump frequency can be computed based on the quantity of jumps and jump duration.

The camera is configured to capture a third user image. Specifically, based on a feature that the camera is easy to move and place, the user may place the camera at a location different from those of both the large-screen device and the mobile phone, to capture a user image from an angle different from those of both the large-screen device and the mobile phone.

In the system provided in this embodiment, the electronic devices may communicate with each other by using wireless communication technologies such as wireless fidelity (wireless fidelity, Wi-Fi), Bluetooth (Bluetooth), and a cellular data service, or may be connected through a physical entity (for example, a USB cable). In this embodiment, user data collected by each electronic device is referred to as data of one dimension. After collecting user data of different dimensions, each electronic device first sends the user data to a central computing device in the system for comprehensive computing, to determine multi-dimensional comprehensive exercise guidance information for the user. Then, the comprehensive exercise guidance information is displayed by a central display device. In this embodiment, the central computing device and the central display device may be the same or different.

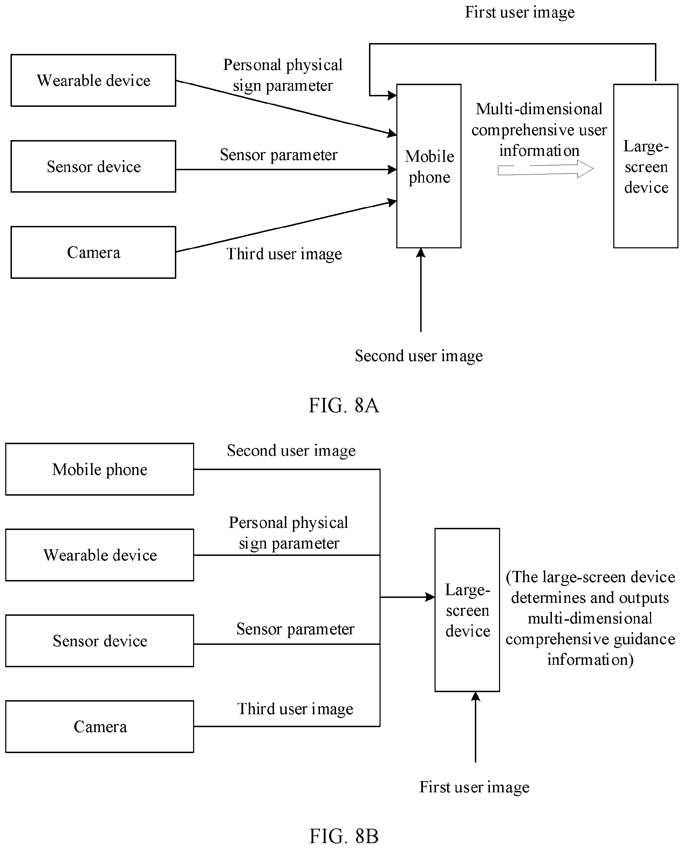

The system shown in FIG. 6 is used as an example. The system includes a large-screen device, a mobile phone, a wearable device, a sensor device, and a camera. In some embodiments, as shown in FIG. 8A, the mobile phone is used as the central computing device to perform comprehensive computing on the user data collected by each electronic device in the system, and the large-screen device is used as the central display device to display exercise guidance information for the user based on a computing result. Compared with the large-screen device, the mobile phone usually has a relatively strong computing capability. Therefore, using the mobile phone as the central computing device can improve a computing rate of the multi-dimensional user data.

In some other embodiments, as shown in FIG. 8B, the large-screen device is used as both the central computing device and the central display device. That is, the smart television needs to perform comprehensive computing on the user data collected by the electronic devices in the system, and further needs to display guidance information for the user based on a computing result. Based on the system provided in this embodiment, data interaction between the electronic devices can be reduced, and resource overheads of the electronic devices can be reduced.

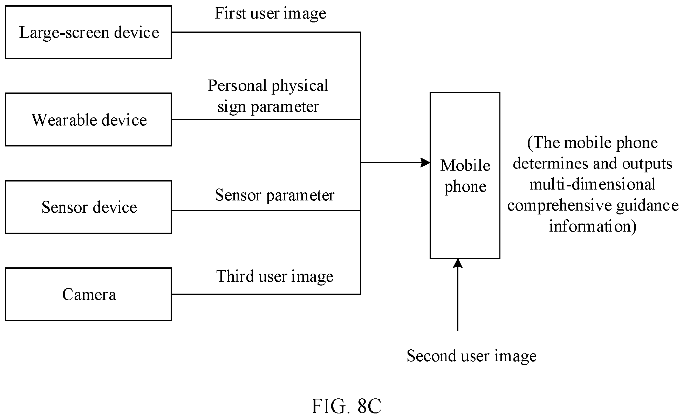

In some other embodiments, as shown in FIG. 8C, the mobile phone is used as both the central computing device and the central display device. That is, the mobile phone needs to perform comprehensive computing on the user data collected by the electronic devices in the system, and further needs to display guidance information for the user based on a computing result. Based on the system provided in this embodiment, data exchange between the electronic devices can be reduced, a computing rate of the multi-dimensional user data can be improved, and resource overheads of the electronic devices can be reduced.

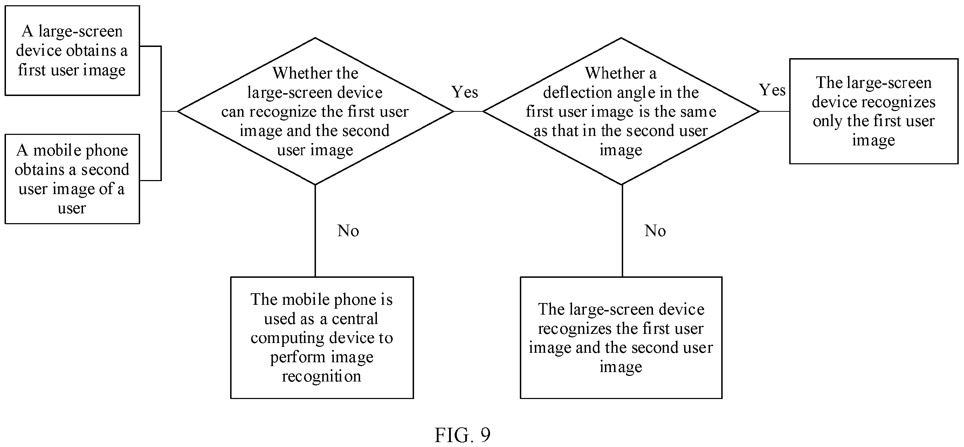

Because a computing capability of the large-screen device is relatively poor compared with that of the mobile phone, when the large-screen device encounters a case in which running freezes, computing takes a long time, user data cannot be recognized, or the like, as shown in FIG. 9, the mobile phone may be set as the central computing device and the central display device, to guide the user to do exercise. If the foregoing case does not occur on the large-screen device, the user data is computed locally. In a computing process, for user images obtained by the central computing device from different electronic devices, for example, the first user image and the second user image, if deflection angles of virtual users in the two images are the same or similar (for example, a difference is within 10°), one of the two images is selected for recognition, to reduce computing pressure of the central computing device and improve computing efficiency. For example, the large-screen device recognizes the first user image to guide the user to do exercise, and the mobile phone assists the large-screen device in recognizing other user data, such as a personal physical sign parameter, a user weight, a jump height, and a jump frequency.

It should be noted that, in this embodiment, when the mobile phone is used as the central computing device or the central display device, the mobile phone may be installed with a same exercise app as the large-screen device, and log in to a same user account, so that in a process of computing the multi-dimensional user data, related computing data, for example, a demonstration image, is directly obtained from an application server.

It may be understood that, when the mobile phone is installed with the exercise app and is used as the central computing device or the central display device, the mobile phone may directly provide exercise guidance for the user in another scenario such as an outdoor scenario, thereby resolving a problem that the user can only do AI workout indoors or needs to do AI workout in front of a smart screen.

The exercise guidance information that can be determined by the system provided in this embodiment includes: item recommendation information before the exercise starts, and an action score, posture correction information, action evaluation information, health prompt information, and the like after the exercise starts.

After turning on the exercise app, the large-screen device can recognize a body type of the user before an exercise item starts, and recommend a corresponding exercise item to the user based on the body type of the user.

In some embodiments, the large-screen device recognizes the body type of the user based on a user image. Specifically, a plurality of standard body type images are pre-stored in the large-screen device. In different standard body type images, deflection angles of a demonstrator are different. Therefore, when recognizing the body type of the user, the large-screen device may first recognize a deflection angle of a virtual user in the user image, and determine a corresponding standard body type image based on the deflection angle. Then, the user image is compared with the corresponding standard body type image, and a deviation between the body type of the user and a standard body type is determined. When the body type of the user is fatter than the standard body type, the smart television may recommend a fat reduction exercise item, for example, an "aerobic exercise", to the user. When the body type of the user is thinner than the standard body type, the smart television may recommend a muscle-building and body-shaping exercise item, for example, "body-shaping yoga" or "overall stretching", to the user.

In some other embodiments, when a body fat measurement apparatus is disposed in the system, the large-screen device may obtain body fat information of the user through the body fat measurement apparatus. The body fat information may include an overall body fat percentage, a local body fat percentage, and the like. Based on the body fat information of the user, the large-screen device may recommend an exercise item suitable for the user to the user. For example, when the overall body fat percentage of the user is higher than a preset value, a fat reduction exercise, for example, an "aerobic exercise", may be recommended to the user. When the body fat percentage of the legs of the user is higher than a preset value, a leg fat reduction item may be recommended to the user. When the body fat percentage of the waist of the user is higher than a preset value, a waist fat reduction item may be recommended to the user.

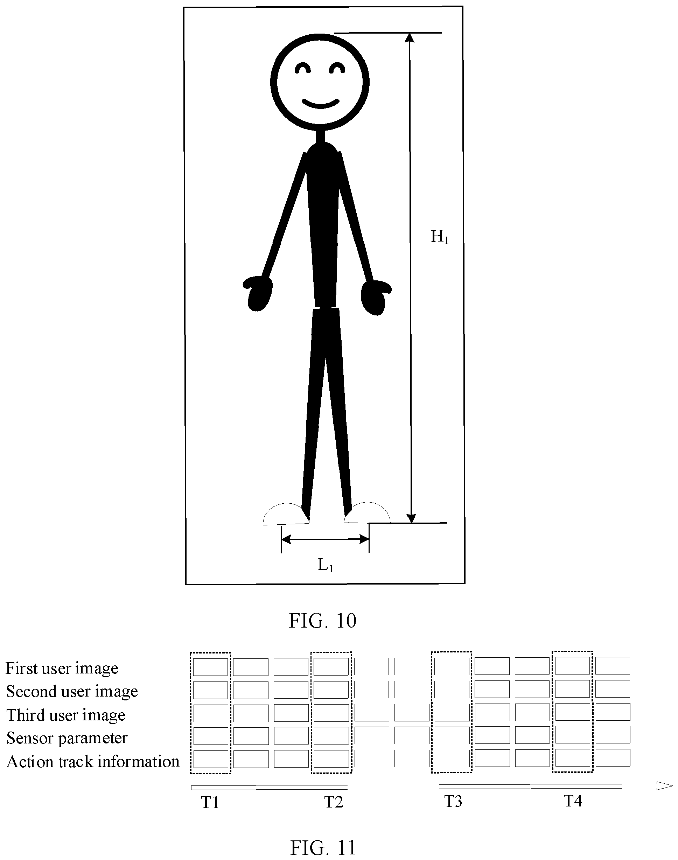

In some other embodiments, as shown in FIG. 10, the large-screen device may capture a user image, and measure a distance L₁ between central locations of two feet of a virtual user in the user image and a height H₁ of the virtual user. Then, a distance L₂ between central locations of two feet of the user is measured based on a parameter fed back by the pressure sensor. Finally, a height H₂ of the user is estimated based on a proportional relationship H₁/L₁ = H₂/L₂. The large-screen device may recommend a corresponding exercise item to the user based on the height. For example, when the height of the user is less than 1.3 meters, it is considered that the user is a child, and an exercise item such as children dance or children gymnastics is recommended to the user.

In some other embodiments, the large-screen device may alternatively recommend an exercise item to the user based on an exercise habit of the user. For example, if the electronic device detects that an exercise item usually done by the user is an "aerobic exercise", the electronic device may preferentially recommend various "aerobic exercise" exercise items to the user.

After the user selects a target exercise item, the large-screen device starts to guide the user to exercise. Because the exercise of the user is a continuous process, as shown in FIG. 11, in a working process of the exercise guidance system, the central computing device needs to determine exercise guidance information once at an interval of a preset time (for example, 0.5s, 1s, or 2s) based on the user data of each dimension. The exercise guidance information includes an action score, an action evaluation icon, posture correction prompt information, user health prompt information, and the like. Descriptions are separately provided below.

### (1) Determining of an action score

In this embodiment, the central computing device may compute an action score of the user based on a user image. The action score may include a phase score and a total score for a user action in a single dimension, and a comprehensive score for a user action in multiple dimensions.

The phase score is a score obtained by the central computing device through computing for user data at a moment in a single dimension, for example, a phase score at a moment T1 or a phase score at a moment T2. The phase score includes a local phase score for a specific body part of the user and an overall phase score for the whole body of the user. The local phase score includes a head phase score, an arm phase score, a waist phase score, a leg phase score, and the like. The overall phase score is an average value of all local phase scores at a same moment (for example, at the moment T1).

The total score indicates an overall exercise status of the user participating in an exercise item, and includes a partial total score and an overall total score. The local total score indicates an exercise status of a part (for example, the head or two arms) of the user in an entire exercise process. The local total score is equal to an average value of all corresponding local phase scores in a corresponding dimension. The overall total score is an average value of all local total scores in a corresponding dimension.

For example, for the first user image captured by the large-screen device, a total score of the arms of the user is equal to an average value of all arm phase scores related to the first user image. The overall total score of the user is equal to an average value of a head total score, an arm total score, a waist total score, and a leg total score that are related to the first user image.

The comprehensive score indicates a comprehensive status of the user action in multiple dimensions. In an example, the comprehensive score is equal to an average value of overall scores in multiple dimensions. For example, the total score is equal to an average value of an overall total score of the front side of the user, an overall total score of the left side of the user, and an overall total score of the back side of the user.

Based on the foregoing description, it can be learned that the overall phase score, the local total score, the overall total score, and the comprehensive score are all obtained through computing based on the local phase score. Therefore, the central computing device computes the local phase score by using an image recognition algorithm, and may determine remaining scores by using a mathematical operation method.

The following describes a manner of determining the local phase score by using an example in which a local phase score of the first user image at a moment T is computed. First, the central computing device may recognize a deflection angle of a virtual user in the first user image by using the ResNet algorithm with reference to the method shown in step S202, and determine a corresponding demonstration image based on the deflection angle. Then, the electronic device recognizes key skeletal nodes of the demonstrator in the demonstration image and key skeletal nodes of the virtual user in the first user image by using a skeletal node algorithm. The key skeletal nodes include skeletal nodes of the head, shoulders, elbows, wrists, crotch, knee joints, ankles, and the like. Finally, location information of the key skeletal nodes of each part of the demonstrator is compared with location information of the key skeletal nodes of each part of the virtual user, and a similarity between an action of the virtual user and a demonstration action is determined based on a location difference, to determine the local phase score of the user at the moment T.

For example, location information of the skeletal nodes of the head of the virtual user is compared with location information of the skeletal nodes of the head of the demonstrator, to determine the head phase score. Location information of the skeletal nodes of the arms (including the skeletal nodes of the shoulders, the elbows, the wrists, and the like) of the virtual user is compared with location information of the skeletal nodes of the arms of the demonstrator, to determine the arm phase score of the user. Location information of the skeletal nodes of the waist (for example, the skeletal nodes of the crotch) of the virtual user is compared with location information of the skeletal nodes of the waist of the demonstrator, to determine the waist phase score of the user. Location information of the skeletal nodes of the legs (for example, the skeletal nodes of knees and ankles of two legs) of the virtual user is compared with location information of the skeletal nodes of the legs of the demonstrator, to determine the two-leg phase score.

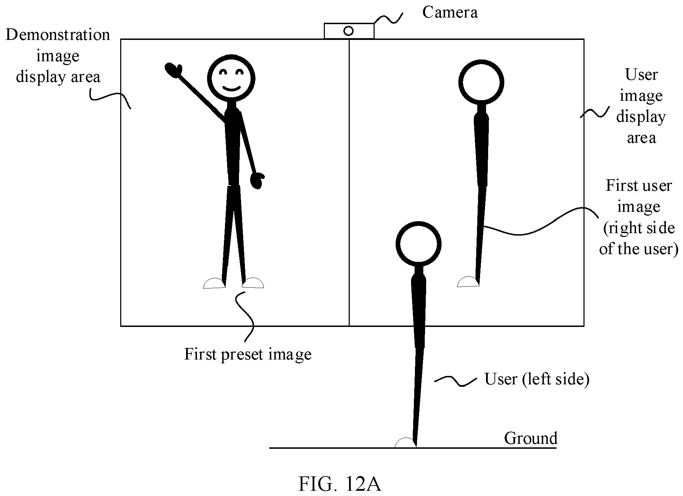

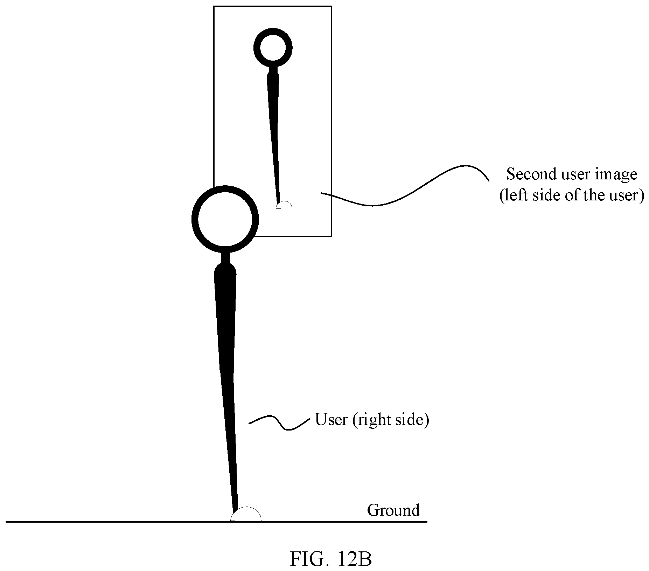

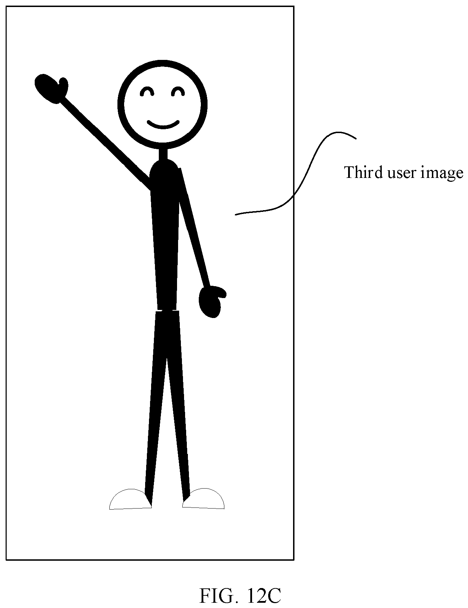

In an example, for the exercise scenario shown in FIG. 6, user images obtained by the central devices are a first user image shown in FIG. 12A (a deflection angle of the virtual user is 90°, and the first user image is a right-side image of the user), a second user image shown in FIG. 12B (a deflection angle of the virtual user is -90°, and the second user image is a left-side image of the user), and a third user image shown in FIG. 12C (a deflection angle of the virtual user is 0°, and the third user image is a front-side image of the user). As shown in Table 1, after recognizing these images, the central computing device may obtain, for example, user action scores shown in Table 1.

**Table 1: User action score table**

| Data collection device | Camera | Mobile phone | Large-screen device |
|---|---|---|---|
| Angle of a user | Front side (deflection angle = 0°) | Left side of the user (deflection angle = 90°) | Left side of the user (deflection angle = - 90°) |
| Total head score | 90 | 85 | 85 |
| Total arm score | 95 | 90 | 95 |
| Total waist score | 85 | 80 | 80 |
| Total two-leg score | 90 | 85 | 80 |
| Total one-side score | 90 | 85 | 85 |
| Comprehensive score | 90 | | |

In addition, in some embodiments, the central computing device may alternatively compare action track information collected by the wearable device with corresponding standard action track information, to recognize a user action and determine various scores of the user action. Alternatively, a shake status of limbs is determined based on an acceleration change that is of the limbs of the user and that is detected by the wearable device, to determine stability of the limbs of the user, and a score of the user action is further determined based on the stability.

In some other embodiments, user actions may be classified into a static action and a transient action based on duration of the user actions. The static action is usually held for a relatively long time, for example, a yoga action or a stretching action. The static action is usually held for a relatively short time, for example, a jump action. For some transient actions, for example, a jump action, the central computing device may further recognize and score the transient actions based on user data collected by the pressure sensor. For example, the central computing device may compare parameters such as a jump height and a jump frequency of the user with parameters such as a standard jump height and a standard jump frequency of the current action, and determine a score of the jump action of the user based on a comparison result.

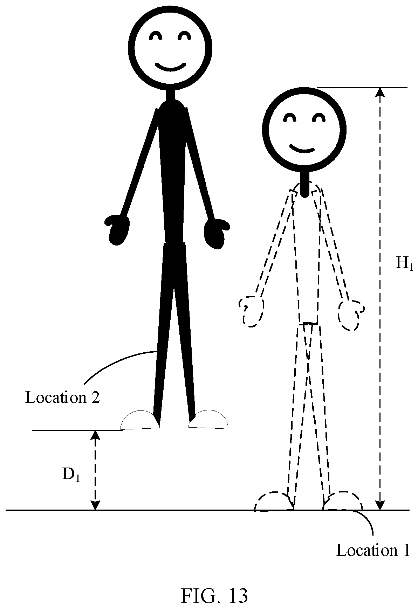

For example, as shown in FIG. 13, when computing the jump height of the user, the central computing device may track and recognize the user, determine a location (namely, a location 1) of the user in the user image before the user jumps, and determine a location 2 of the user in the user image when the user jumps to a highest point, to determine a height difference D₁ between the location 1 and the location 2 in the user image. Then, the jump height D₂ of the user is determined based on a proportional relationship D₁ /D₂ = H₁ /H₂. H, is a height of the virtual user in the user image, and H₂ is an estimated value of a real height of the user. Compared with an existing exercise guidance system, the system provided in this embodiment has a stronger capability of recognizing and analyzing a transient action.

After determining the various scores of the user action, the central computing device sends the various scores to the central display device, and the central display device performs related display.

In some embodiments, the large-screen device may display action evaluation information at an interval of a preset time based on an overall phase score of the user action. For example, when the overall phase score of the user is higher than 90, the large-screen device displays a prompt of action evaluation information such as "Perfect" or "Excellent". When the overall phase score of the user is between 80 and 90, a prompt of action evaluation information such as "Come on" or "Keep trying" is intelligently displayed.

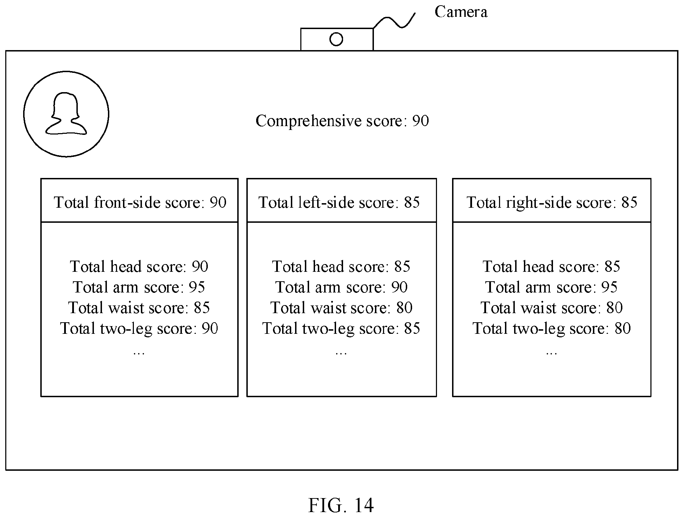

In some other embodiments, as shown in FIG. 14, after running of an exercise item ends, the smart television may display a comprehensive score of the exercise item done by the user, an overall total score of a single dimension (for example, a front-side total score, a left-side total score, and a right-side total score), and a local total score (for example, a head total score, an arm total score, a waist total score, and a two-leg total score) of the user for the user action on each side. Based on the total scores of different parts, the user can determine an exercise status of each part of the body in the exercise process, and analyze a workout action and an exercise status in more detail. Based on the comprehensive score, the user can learn about a comprehensive exercise status of the user.

### (2) Posture correction prompt information

In a possible implementation, when detecting that a local phase score (for example, an arm phase score) of a user action is less than a preset value, the central computing device determines a location difference between an arm of the virtual user and an arm of the demonstrator, and displays posture correction information based on the location difference, to prompt the user to adjust an exercise posture. For example, when the arm of the virtual user is lower than the arm of the demonstrator, the central computing device may determine the posture correction prompt information as "Please raise the arm". After determining the posture correction prompt information, the central computing device may send the posture correction prompt information to the central display device for display.

In another possible implementation, the central computing device also compares a distance between two feet of the user with a standard distance that is between the two feet and that corresponds to the current action, to determine whether the two feet of the user are in a standard action, for example, whether a distance between two legs of the user is excessively long or whether the distance between the two legs of the user is not enough, and displays corresponding prompt information to remind the user to perform adjustment.

In some other embodiments, for some jumping exercises (for example, a rope skipping exercise), the central computing device may compare a jump frequency of the user with a standard jump frequency of the current action. When the jump frequency of the user is higher than a jump frequency preset value (for example, 15%), the central computing device controls the central display device to prompt the user to reduce the jump frequency, to reduce exercise intensity. When the jump frequency of the user is lower than a jump frequency preset value (for example, 15%), the central computing device controls the central display device to prompt the user to increase the jump frequency, to increase exercise intensity.

In addition, the central devices may further correct a jump height of the user. For example, when the jump height of the user is higher than a standard jump height preset value (for example, 15%), the central computing device controls the central display device to prompt the user to reduce the jump height, to reduce exercise intensity and save physical strength. When the jump height of the user is higher than a standard jump height preset value (for example, 15%), the central computing device controls the central display device to prompt the user to increase the jump height, to increase exercise intensity.

### (3) Health prompt information

In this embodiment, the central computing device may determine health prompt information based on the personal physical sign parameter collected by the wearable device, and the health prompt information is displayed by the central display device, to indicate whether the user can do exercise currently. For example, when the heart rate of the user is greater than 160 times/minute, the user is prompted with "The heart rate is too fast, please take a rest before exercising". Alternatively, when systolic blood pressure of the user is greater than 160 mmHg, or diastolic blood pressure is greater than 120 mmHg, the user is prompted, by the smart television, with "The blood pressure is too high, please take a rest before exercising".

In some other embodiments, the central computing device may determine the exercise intensity of the user based on the heart rate of the user. For example, when the heart rate of the user is 60% HRmax or below, it is determined that an exercise intensity level of the user is "low intensity". When the heart rate of the user is between 60% HRmax and 80% HRmax, it is determined that an exercise intensity level of the user is "medium intensity". When the heart rate of the user is 80% HRmax or above, it is determined that an exercise intensity level of the user is "high intensity". HRmax is a maximum value of the heart rate of the user. Generally, HRmax = 220 - User age, where the user age is determined by a value entered by the user. For example, if the user is 20 years old, HRmax of the user is as follows: HRmax = 220 - 20 = 200. When detecting that the exercise intensity of the user is equal to or higher than an intensity level of the exercise item, the electronic device reminds the user to reduce the exercise intensity.

In addition, when the smart television or the mobile phone obtains user images photographed at a plurality of different angles, the smart television or the mobile phone may establish a three-dimensional exercise model for the user with reference to the user images photographed at the different angles. The three-dimensional exercise model can display a part or all of a process in which the user participates in the exercise item. When the electronic device displays the three-dimensional model, the electronic device may control, based on a user instruction, the three-dimensional model to rotate, to display an exercise posture of the user from different angles.

In conclusion, the exercise guidance system provided in this embodiment of this application includes at least two electronic devices. The two electronic devices can collect multi-dimensional user data, and guide, based on the multi-dimensional user data, the user to do exercise. Compared with a case in which exercise guidance is performed on a user only based on single-dimensional user data collected from a single source, the system provided in this embodiment can improve accuracy of exercise guidance, for example, reduce a case in which an action score is inaccurate.

It should be understood that sequence numbers of the steps do not mean an execution sequence in the foregoing embodiments. The execution sequence of the processes should be determined based on functions and internal logic of the processes, and should not be construed as any limitation on the implementation processes of embodiments of this application.

An embodiment of this application further provides an electronic device. The electronic device is configured to perform a method for determining user information provided in the foregoing embodiment. After determining a deflection angle of a user image, the electronic device provided in this embodiment of this application compares the user image with a second preset image corresponding to the deflection angle, to determine exercise guidance information for a user, so that accuracy of user action recognition can be improved. On this basis, in a process of doing exercise, the user does not need to be limited by the deflection angle, and can select, based on a preference of the user, a deflection angle relative to the electronic device. This provides better user experience.

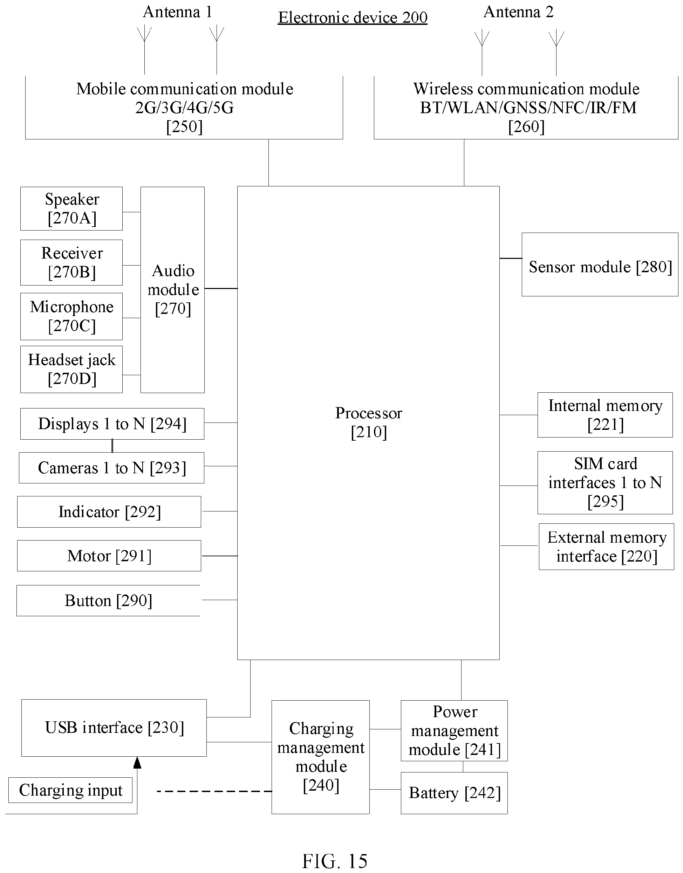

FIG. 15 is a schematic diagram of a structure of an electronic device. The electronic device 200 may include a processor 210, an external memory interface 220, an internal memory 221, a universal serial bus (universal serial bus, USB) interface 230, a charging management module 240, a power management module 241, a battery 242, an antenna 1, an antenna 2, a mobile communication module 250, a wireless communication module 260, an audio module 270, a speaker 270A, a receiver 270B, a microphone 270C, a headset jack 270D, a sensor module 280, a button 290, a motor 291, an indicator 292, a camera 293, a display 294, a subscriber identification module (subscriber identification module, SIM) card interface 295, and the like.

It may be understood that the structure shown in this embodiment of this application does not constitute a specific limitation on the electronic device 200. In some other embodiments of this application, the electronic device 200 may include more components or fewer components than those shown in the figure, or some components may be combined, or some components may be separated, or a different component deployment may be used. The components shown in the figure may be implemented in hardware, software, or a combination of software and hardware.

For example, when the electronic device 200 is a mobile phone or a tablet computer, the electronic device 200 may include all components shown in the figure, or may include only some components shown in the figure.

For example, when the electronic device 200 is a large-screen device such as a smart television or a smart screen, the electronic device 200 may include all components shown in the figure, or may include only some components shown in the figure.

The processor 210 may include one or more processing units. For example, the processor 210 may include an application processor (application processor, AP), a modem processor, a graphics processing unit (graphics processing unit, GPU), an image signal processor (image signal processor, ISP), a controller, a memory, a video codec, a digital signal processor (digital signal processor, DSP), a baseband processor, a neural-network processing unit (neural-network processing unit, NPU), and/or the like. Different processing units may be independent components, or may be integrated into one or more processors.

The controller may be a nerve center and a command center of the electronic device 200. The controller may generate an operation control signal based on an instruction operation code and a time sequence signal, to complete control of instruction reading and instruction execution.

A memory may be further disposed in the processor 210, and is configured to store instructions and data. In some embodiments, the memory in the processor 210 is a cache memory. The memory may store instructions or data just used or cyclically used by the processor 210. If the processor 210 needs to use the instructions or the data again, the processor 210 may directly invoke the instructions or the data from the memory. This avoids repeated access, reduces waiting time of the processor 210, and improves system efficiency.

In some embodiments, the processor 210 may include one or more interfaces. The interface may include an inter-integrated circuit (inter-integrated circuit, I2C) interface, an inter-integrated circuit sound (inter-integrated circuit sound, I2S) interface, a pulse code modulation (pulse code modulation, PCM) interface, a universal asynchronous receiver/transmitter (universal asynchronous receiver/transmitter, UART) interface, a mobile industry processor interface (mobile industry processor interface, MIPI), a general-purpose input/output (general-purpose input/output, GPIO) interface, a subscriber identity module (subscriber identity module, SIM) interface, a universal serial bus (universal serial bus, USB) interface, and/or the like.

The I2C interface is a two-way synchronous serial bus, and includes a serial data line (serial data line, SDA) and a serial clock line (serial clock line, SCL). In some embodiments, the processor 210 may include a plurality of groups of I2C buses. The processor 210 may be separately coupled to a charger, a flash, the camera 293, and the like through different I2C bus interfaces.

The I2S interface may be configured to perform audio communication. In some embodiments, the processor 210 may include a plurality of groups of I2S buses. The processor 210 may be coupled to the audio module 270 through the I2S bus, to implement communication between the processor 210 and the audio module 270. In some embodiments, the audio module 270 may transmit an audio signal to the wireless communication module 260 through the I2S interface.

The PCM interface may also be configured to: perform audio communication, and sample, quantize, and encode an analog signal. In some embodiments, the audio module 270 may be coupled to the wireless communication module 260 through a PCM bus interface.

In some embodiments, the audio module 270 may also transmit an audio signal to the wireless communication module 260 through the PCM interface. Both the I2S interface and the PCM interface may be configured to perform audio communication.

The UART interface is a universal serial data bus, and is configured to perform asynchronous communication. The bus may be a two-way communication bus. The bus converts to-be-transmitted data between serial communication and parallel communication.

In some embodiments, the UART interface is generally configured to connect the processor 210 and the wireless communication module 260. For example, the processor 210 communicates with a Bluetooth module in the wireless communication module 260 through the UART interface, to implement a Bluetooth function. In some embodiments, the audio module 270 may transmit an audio signal to the wireless communication module 260 through the UART interface, to implement a function of playing music through a Bluetooth headset.

The MIPI interface may be configured to connect the processor 210 and a peripheral device such as the display 294 or the camera 293. The MIPI interface includes a camera serial interface (camera serial interface, CSI), a display serial interface (display serial interface, DSI), and the like. In some embodiments, the processor 210 communicates with the camera 293 through the CSI interface, to implement a photographing function of the electronic device 200. The processor 210 communicates with the display 294 through the DSI interface, to implement a display function of the electronic device 200.

The GPIO interface may be configured by software. The GPIO interface may be configured as a control signal or a data signal. In some embodiments, the GPIO interface may be configured to connect the processor 210 to the camera 293, the display 294, the wireless communication module 260, the audio module 270, the sensor module 280, or the like. The GPIO interface may alternatively be configured as an I2C interface, an I2S interface, a UART interface, an MIPI interface, or the like.

The USB interface 230 is an interface that conforms to a USB standard specification, and may be specifically a mini USB interface, a micro USB interface, a USB Type-C interface, or the like. The USB interface 230 may be configured to connect to a charger to charge the electronic device 200, or may be configured to transmit data between the electronic device 200 and a peripheral device. Alternatively, the USB interface 230 may be configured to connect to a headset, to play audio through the headset. The interface may be alternatively configured to connect to another electronic device, for example, an AR device.

It may be understood that an interface connection relationship between the modules shown in embodiments of this application is merely an example for description, and does not constitute a limitation on the structure of the electronic device 200. In some other embodiments of this application, the electronic device 200 may alternatively use an interface connection manner different from that in the foregoing embodiment, or a combination of a plurality of interface connection manners.

The charging management module 240 is configured to receive a charging input from a charger. The charger may be a wireless charger or a wired charger. In some embodiments of wired charging, the charging management module 240 may receive a charging input from the wired charger through the USB interface 230. In some embodiments of wireless charging, the charging management module 240 may receive a wireless charging input through a wireless charging coil of the electronic device 200. The charging management module 240 may also supply power to the electronic device through the power management module 241 while charging the battery 242.

The power management module 241 is configured to connect to the battery 242, the charging management module 240, and the processor 210. The power management module 241 receives an input of the battery 242 and/or the charging management module 240, to supply power to the processor 210, the internal memory 221, the external memory, the display 294, the camera 293, the wireless communication module 260, and the like. The power management module 241 may be further configured to monitor parameters such as a battery capacity, a battery cycle count, and a battery health status (electric leakage or impedance).

In some other embodiments, the power management module 241 may be alternatively disposed in the processor 210. In some other embodiments, the power management module 241 and the charging management module 240 may be alternatively disposed in a same device.

A wireless communication function of the electronic device 200 may be implemented through the antenna 1, the antenna 2, the mobile communication module 250, the wireless communication module 260, the modem processor, the baseband processor, and the like.

The antenna 1 and the antenna 2 are configured to transmit and receive an electromagnetic wave signal. Each antenna in the electronic device 200 may be configured to cover one or more communication frequency bands. Different antennas may be further multiplexed, to improve antenna utilization. For example, the antenna 1 may be multiplexed as a diversity antenna of a wireless local area network. In some other embodiments, the antenna may be used in combination with a tuning switch.

The mobile communication module 250 may provide a solution that includes wireless communication such as 2G/3G/4G/5G and that is applied to the electronic device 200. The mobile communication module 250 may include at least one filter, a switch, a power amplifier, a low noise amplifier (low noise amplifier, LNA), and the like. The mobile communication module 250 may receive an electromagnetic wave through the antenna 1, perform processing such as filtering or amplification on the received electromagnetic wave, and transmit the electromagnetic wave to the modem processor for demodulation. The mobile communication module 250 may further amplify a signal modulated by the modem processor, and convert the signal into an electromagnetic wave for radiation through the antenna 1.

In some embodiments, at least some function modules of the mobile communication module 250 may be disposed in the processor 210. In some embodiments, at least some function modules of the mobile communication module 250 may be disposed in a same device as at least some modules of the processor 210.

The modem processor may include a modulator and a demodulator. The modulator is configured to modulate a to-be-sent low-frequency baseband signal into a medium-high frequency signal. The demodulator is configured to demodulate a received electromagnetic wave signal into a low-frequency baseband signal. Then, the demodulator transmits the low-frequency baseband signal obtained through demodulation to the baseband processor for processing. The low-frequency baseband signal is processed by the baseband processor and then transmitted to the application processor. The application processor outputs a sound signal through an audio device (which is not limited to the speaker 270A, the receiver 270B, or the like), or displays an image or a video through the display 294. In some embodiments, the modem processor may be an independent device. In some other embodiments, the modem processor may be independent of the processor 210, and is disposed in a same device as the mobile communication module 250 or another function module.

The wireless communication module 260 may provide a wireless communication solution that is applied to the electronic device 200 and that includes a wireless local area network (wireless local area network, WLAN) (for example, a wireless fidelity (wireless fidelity, Wi-Fi) network), Bluetooth (Bluetooth, BT), a global navigation satellite system (global navigation satellite system, GNSS), frequency modulation (frequency modulation, FM), a near field communication (near field communication, NFC) technology, an infrared (infrared, IR) technology, and the like. The wireless communication module 260 may be one or more devices integrating at least one communication processing module. The wireless communication module 260 receives an electromagnetic wave through the antenna 2, performs frequency modulation and filtering processing on an electromagnetic wave signal, and sends a processed signal to the processor 210. The wireless communication module 260 may further receive a to-be-sent signal from the processor 210, perform frequency modulation and amplification on the signal, and convert the signal into an electromagnetic wave for radiation through the antenna 2.

In some embodiments, the antenna 1 and the mobile communication module 250 in the electronic device 200 are coupled, and the antenna 2 and the wireless communication module 260 in the electronic device 200 are coupled, so that the electronic device 200 can communicate with a network and another device through a wireless communication technology. The wireless communication technology may include a global system for mobile communications (global system for mobile communications, GSM), a general packet radio service (general packet radio service, GPRS), code division multiple access (code division multiple access, CDMA), wideband code division multiple access (wideband code division multiple access, WCDMA), time-division code division multiple access (time-division code division multiple access, TD-SCDMA), long term evolution (long term evolution, LTE), BT, a GNSS, a WLAN, NFC, FM, an IR technology, and/or the like. The GNSS may include a global positioning system (global positioning system, GPS), a global navigation satellite system (global navigation satellite system, GLONASS), a BeiDou navigation satellite system (BeiDou navigation satellite system, BDS), a quasi-zenith satellite system (quasi-zenith satellite system, QZSS), and/or a satellite based augmentation system (satellite based augmentation system, SBAS).

The electronic device 200 implements a display function through the GPU, the display 294, the application processor, and the like. The GPU is a microprocessor for image processing, and is connected to the display 294 and the application processor. The GPU is configured to: perform mathematical and geometric computing, and render an image. The processor 210 may include one or more GPUs that execute program instructions to generate or change display information.

The display 294 is configured to display an image, a video, and the like. For example, a teaching video and a user action image video in this embodiment of this application. The display 294 includes a display panel. The display panel may be a liquid crystal display (liquid crystal display, LCD), an organic light-emitting diode (organic light-emitting diode, OLED), an active-matrix organic light emitting diode (active-matrix organic light emitting diode, AMOLED), a flexible light-emitting diode (flex light-emitting diode, FLED), a mini-LED, a micro-LED, a micro-OLED, a quantum dot light emitting diode (quantum dot light emitting diode, QLED), or the like. In some embodiments, the electronic device 200 may include one or N displays 294. N is a positive integer greater than 1.

The electronic device 200 may implement a photographing function through the ISP, the camera 293, the video codec, the GPU, the display 294, the application processor, and the like.

The ISP is configured to process data fed back by the camera 293. For example, during photographing, a shutter is pressed, and light is transmitted to a photosensitive element of the camera through a lens. An optical signal is converted into an electrical signal, and the photosensitive element of the camera transmits the electrical signal to the ISP for processing, to convert the electrical signal into a visible image. The ISP may further perform algorithm optimization on noise, brightness, and complexion of the image. The ISP may further optimize parameters such as exposure and a color temperature of a photographing scenario. In some embodiments, the ISP may be disposed in the camera 293.

The camera 293 is configured to capture a static image or a video. An optical image of an object is generated through the lens, and is projected onto the photosensitive element. The photosensitive element may be a charge coupled device (charge coupled device, CCD) or a complementary metal-oxide-semiconductor (complementary metal-oxide-semiconductor, CMOS) phototransistor. The photosensitive element converts an optical signal into an electrical signal, and then transmits the electrical signal to the ISP to convert the electrical signal into a digital image signal. The ISP outputs the digital image signal to the DSP for processing. The DSP converts the digital image signal into an image signal in a standard format such as RGB or YUV. In some embodiments, the electronic device 200 may include one or N cameras 293. N is a positive integer greater than 1.

The digital signal processor is configured to process a digital signal, and may process another digital signal in addition to the digital image signal. For example, when the electronic device 200 selects a frequency, the digital signal processor is configured to perform Fourier transform or the like on frequency energy.

The video codec is configured to compress or decompress a digital video. The electronic device 200 may support one or more types of video codecs. In this way, the electronic device 200 may play or record videos in a plurality of coding formats, for example, moving picture experts group (moving picture experts group, MPEG)-1, MPEG-2, MPEG-3, and MPEG-4.

The NPU is a neural-network (neural-network, NN) computing processor, quickly processes input information by referring to a structure of a biological neural network, for example, by referring to a mode of transmission between human brain neurons, and may further continuously perform self-learning. The NPU may implement applications such as intelligent cognition of the electronic device 200, for example, image recognition, facial recognition, speech recognition, and text understanding.

In this embodiment of this application, the NPU or another processor may be configured to perform operations such as analysis and processing on an image in a video stored in the electronic device 200.

The external memory interface 220 may be configured to connect to an external memory card, for example, a micro SD card, to extend a storage capability of the electronic device 200. The external memory card communicates with the processor 210 through the external memory interface 220, to implement a data storage function. For example, files such as music and videos are stored in the external memory card.

The internal memory 221 may be configured to store computer-executable program code. The executable program code includes instructions. The processor 210 runs the instructions stored in the internal memory 221, to perform various function applications and data processing of the electronic device 200. The internal memory 221 may include a program storage area and a data storage area. The program storage area may store an operating system, an application required for at least one function (such as a sound playing function and an image display function). The data storage area may store data (such as audio data and a phone book) created during use of the electronic device 200.

In addition, the internal memory 221 may include a high-speed random access memory, and may further include a non-volatile memory, for example, at least one magnetic disk storage device, a flash memory device, or a universal flash storage (universal flash storage, UFS).

The electronic device 200 may implement an audio function through the audio module 270, the speaker 270A, the receiver 270B, the microphone 270C, the headset jack 270D, the application processor, and the like.

The audio module 270 is configured to convert a digital audio signal into an analog audio signal for output, and is further configured to convert an analog audio input into a digital audio signal. The audio module 270 may be further configured to code and decode an audio signal. In some embodiments, the audio module 270 may be disposed in the processor 210, or some function modules in the audio module 270 are disposed in the processor 210.

The speaker 270A, also referred to as a "loudspeaker", is configured to convert an audio electrical signal into a sound signal. The electronic device 200 may receive music or receive a hands-free call through the loudspeaker 270A. For example, the speaker may play a comparison analysis result provided in this embodiment of this application.

The receiver 270B, also referred to as an "earpiece", is configured to convert an audio electrical signal into a sound signal. When a call is answered or voice information is received by the electronic device 200, the receiver 270B may be placed close to a human ear to listen to a voice.

The microphone 270C, also referred to as a "mike" or a "mic", is configured to convert a sound signal into an electrical signal. When making a call or sending voice information, a user may make a sound by moving a human mouth close to the microphone 270C to input a sound signal to the microphone 270C. At least one microphone 270C may be disposed in the electronic device 200. In some other embodiments, two microphones 270C may be disposed in the electronic device 200, to collect a sound signal and further implement a noise reduction function. In some other embodiments, three, four, or more microphones 270C may be alternatively disposed in the electronic device 200, to collect a sound signal, reduce noise, identify a sound source, implement a directional recording function, and the like.

The headset jack 270D is configured to connect to a wired headset. The headset jack 270D may be the USB interface 230, or may be a 3.5 mm open mobile terminal platform (open mobile terminal platform, OMTP) standard interface or a cellular telecommunications industry association of the USA (cellular telecommunications industry association of the USA, CTIA) standard interface.

The button 290 includes a power button, a volume button, and the like. The button 290 may be a mechanical button, or may be a touch button. The electronic device 200 may receive a button input, and generate a button signal input related to a user setting and function control of the electronic device 200.

The motor 291 may generate a vibration prompt. The motor 291 may be configured to provide an incoming call vibration prompt and a touch vibration feedback. For example, touch operations performed on different applications (for example, a photographing application and an audio playing application) may correspond to different vibration feedback effect. The motor 291 may also correspond to different vibration feedback effect for touch operations performed on different areas of the display 294. Different application scenarios (for example, a time reminder, information receiving, an alarm clock, and a game) may also correspond to different vibration feedback effect. Touch vibration feedback effect may be further customized.

The indicator 292 may be an indicator light, and may be configured to indicate a charging status and a power change, or may be configured to indicate a message, a missed call, a notification, and the like.

The SIM card interface 295 is configured to connect to a SIM card. The SIM card may be inserted into the SIM card interface 295 or removed from the SIM card interface 295, to implement contact with or separation from the electronic device 200. The electronic device 200 may support one or N SIM card interfaces, where N is a positive integer greater than 1. The SIM card interface 295 may support a nano-SIM card, a micro-SIM card, a SIM card, and the like. A plurality of cards may be simultaneously inserted into a same SIM card interface 295. The plurality of cards may be of a same type or different types. The SIM card interface 295 may also be compatible with different types of SIM cards. The SIM card interface 295 may also be compatible with an external memory card. The electronic device 200 interacts with a network through the SIM card to implement functions such as calling and data communication. In some embodiments, the electronic device 200 uses an eSIM, namely, an embedded SIM card. The eSIM card may be embedded into the electronic device 200, and cannot be separated from the electronic device 200.

An embodiment of this application provides a chip system. The chip system includes a memory and a processor. The processor executes a computer program stored in the memory, to implement the method for determining exercise guidance information provided in the foregoing embodiments. The chip system may be a single chip or a chip module including a plurality of chips.

An embodiment of this application provides a computer-readable storage medium. The computer-readable storage medium stores a computer program. When the computer program is executed by a processor, the method for determining exercise guidance information provided in the foregoing embodiments is implemented.

It should be understood that the processor in embodiments of this application may be a central processing unit (central processing unit, CPU), another general-purpose processor, a digital signal processor (digital signal processor, DSP), an application specific integrated circuit (application specific integrated circuit, ASIC), a field programmable gate array (field programmable gate array, FPGA) or another programmable logic device, a discrete gate or transistor logic device, a discrete hardware component, or the like. The general-purpose processor may be a microprocessor, or the processor may be any conventional processor, or the like.

It may be understood that the memory in embodiments of this application may be a volatile memory or a non-volatile memory, or may include a volatile memory and a non-volatile memory. The non-volatile memory may be a read-only memory (read-only memory, ROM), a programmable read-only memory (programmable ROM, PROM), an erasable programmable read-only memory (erasable PROM, EPROM), an electrically erasable programmable read-only memory (electrically EPROM, EEPROM), or a flash memory. The volatile memory may be a random access memory (random access memory, RAM), and is used as an external cache. Based on description used as an example instead of a limitation, many forms of RAMs may be used, for example, a static random access memory (static RAM, SRAM), a dynamic random access memory (dynamic RAM, DRAM), a synchronous dynamic random access memory (synchronous DRAM, SDRAM), a double data rate synchronous dynamic random access memory (double data rate SDRAM, DDR SDRAM), an enhanced synchronous dynamic random access memory (enhanced SDRAM, ESDRAM), a synchlink dynamic random access memory (synchlink DRAM, SLDRAM), and a direct rambus random access memory (direct rambus RAM, DR RAM).

Persons skilled in the art may clearly understand that, for the purpose of convenient and brief description, for a detailed working process of the foregoing system, apparatus, and unit, refer to a corresponding process in the foregoing method embodiments, and details are not described herein again.

In descriptions of embodiments of this application, unless otherwise specified, "/" means "or". For example, A/B may indicate A or B. The term "and/or" in this specification merely describes an association relationship between associated objects, and indicates that three relationships may exist. For example, A and/or B may indicate the following three cases: Only A exists, both A and B exist, and only B exists. In addition, in the descriptions in embodiments of this application, "a plurality of" means two or more.

The terms "first" and "second" mentioned above are merely intended for a purpose of description, and shall not be understood as an indication or implication of relative importance or implicit indication of a quantity of indicated technical features. Therefore, a feature limited by "first" or "second" may explicitly or implicitly include one or more features. In the descriptions of embodiments, unless otherwise specified, "a plurality of" means two or more.

Reference to "an embodiment", "some embodiments", or the like described in the specification of this application indicates that one or more embodiments of this application include a specific feature, structure, or characteristic described with reference to the embodiments. Therefore, statements such as "in an embodiment" "in some embodiments" "in some other embodiments" and "in other embodiments" that appear at different places in this specification do not necessarily refer to a same embodiment. Instead, the statements mean "one or more but not all of embodiments", unless otherwise specifically emphasized in another manner. The terms "include", "comprise", "have", and their variants all mean "include but are not limited to", unless otherwise specifically emphasized in another manner.

The foregoing embodiments are merely intended to describe the technical solutions of this application, but not to limit this application. Although this application is described in detail with reference to the foregoing embodiments, persons of ordinary skill in the art should understand that they may still make modifications to the technical solutions described in the foregoing embodiments or make equivalent replacements to some technical features thereof, without departing from the spirit and scope of the technical solutions of embodiments of this application, all of which shall fall within the protection scope of this application.

## Claims

1. A method for determining exercise guidance information, applied to an electronic device, wherein the method comprises:
displaying a first preset image and a captured user image;
recognizing a deflection angle of a virtual user in the user image;
determining, from a demonstration action set, a second preset image corresponding to the deflection angle, wherein preset images corresponding to different deflection angles are stored in the demonstration action set; and
determining exercise guidance information for a user based on the user image and the second preset image, wherein
the first preset image is the same as or different from the second preset image.

2. The method according to claim 1, wherein the method further comprises:
displaying the second preset image while displaying the first preset image; or
replacing the first preset image with the second preset image.

3. The method according to claim 1 or 2, wherein the exercise guidance information comprises at least one of an action score, an action comment, an action evaluation icon, posture correction information, consumed calories, and exercise duration.

4. An electronic device, wherein the electronic device is configured to:
display a first preset image and a captured user image;
recognize a deflection angle of a virtual user in the user image;
determine, from a demonstration action set, a second preset image corresponding to the deflection angle, wherein preset images corresponding to different deflection angles are stored in the demonstration action set; and
determine exercise guidance information for a user based on the user image and the second preset image, wherein
the first preset image is the same as or different from the second preset image.

5. The electronic device according to claim 4, wherein the electronic device is further configured to:
display the second preset image while displaying the first preset image; or
replace the first preset image with the second preset image.

6. The electronic device according to claim 3 or 4, wherein the exercise guidance information comprises at least one of an action score, an action comment, an action evaluation icon, posture correction information, consumed calories, and exercise duration.

7. An exercise guidance system, comprising a first electronic device and at least one second electronic device, wherein the first electronic device is configured to perform the method according to any one of claims 1 to 3; and
the first electronic device determines exercise guidance information for a user based on both locally collected user data and user data collected by the at least one second electronic device, wherein
the at least one second electronic device is the same or different.

8. The system according to claim 7, wherein the user data collected by the first electronic device is a first user image, and the user data collected by the second electronic device is a second user image; and a deflection angle of a virtual user in the first user image is different from that in the second user image.

9. The system according to claim 7 or 8, wherein
the user data collected by the second electronic device is body fat information; and
the first electronic device recommends a corresponding exercise item to the user based on the body fat information.

10. The system according to any one of claims 7 to 9, wherein
the user data collected by the second electronic device is a personal physical sign parameter; and
the first electronic device displays health prompt information based on the personal physical sign parameter, wherein the health prompt information indicates whether the user can do exercise.

11. The system according to claim 10, wherein the personal physical sign parameter comprises at least one of a heart rate, blood pressure, body temperature, and blood oxygen saturation.

12. The system according to claim 11, wherein the user data collected by the second electronic device comprises a jump height and a jump frequency of the user.

13. The system according to any one of claims 7 to 12, wherein the first electronic device is connected to the at least one second electronic device through a far field communication technology, a near field communication technology, or a physical entity.

14. A chip system, wherein the chip system comprises a memory and a processor, and the processor executes a computer program stored in the memory, to implement the method according to any one of claims 1 to 3.

15. A computer-readable storage medium, wherein the computer-readable storage medium stores a computer program, and when the computer program is executed by a processor, the method according to any one of claims 1 to 3 is implemented.
